(19) Europäisches Patentamt
European Patent Office
Office européen des brevets

(11) Veröffentlichungsnummer: **0 216 731**
**B1**

(12) **EUROPÄISCHE PATENTSCHRIFT**

(45) Veröffentlichungstag der Patentschrift: **14.03.90**

(21) Anmeldenummer: **86810405.0**

(22) Anmeldetag: **08.09.86**

(51) Int. Cl.$^5$: **C 07 H 17/08,** C 07 D 493/22, A 61 K 31/70, A 61 K 31/365, C 12 P 19/62, A 01 N 43/90 // (C07D493/22, 323:00, 311:00, 311:00, 311:00, 307:00),(C12P19/62, C12R1:00)

# (54) Makrozyklische Antibiotika.

(30) Priorität: **13.09.85 CH 3986/85**

(43) Veröffentlichungstag der Anmeldung: **01.04.87 Patentblatt 87/14**

(45) Bekanntmachung des Hinweises auf die Patenterteilung: **14.03.90 Patentblatt 90/11**

(84) Benannte Vertragsstaaten: **AT BE CH DE FR GB IT LI LU NL SE**

(56) Entgegenhaltungen:

**TETRAHEDRON LETTERS, Nr. 40, 1966, Seiten 4867-4870, Pergamon Press Ltd., GB; O.E. EDWARDS et al.: "N-dioxides, a new class of amine oxides"**

**TETRAHEDRON LETTERS, Band 26, Nr. 49, 1985, Seiten 6031-6034, Pergamon Press Ltd., GB; R. JANSEN et al.: "Isolation and spectroscopic structure elucidation of sorangicin A, a new type of macrolide-polyether antibiotic from gliding bacteria"**

(73) Patentinhaber: **Gesellschaft für Biotechnologische Forschung mbH (GBF)
Mascheroder Weg 1
D-3300 Braunschweig-Stöckheim (DE)**

(72) Erfinder: **Reichenbach, Hans, Prof. Dr.
Platanenstrasse 35
D-3340 Wolfenbüttel (DE)**
Erfinder: **Höfle, Gerhard, Prof. Dr.
Am Windmühlenberg 2
D-3300 Braunschweig (DE)**
Erfinder: **Augustiniak, Hermann, Dr.
In den Lindendöhren 19
D-3340 Wolfenbüttel (DE)**
Erfinder: **Bedorf, Norbert Dr.
Krukenbergstrasse 4
D-3307 Königslutter (DE)**
Erfinder: **Gerth, Klaus, Dr.
Am Butterbusch 17
D-3300 Braunschweig (DE)**
Erfinder: **Irschik, Herbert, Dr.
Masurenweg 15
D-3340 Wolfenbüttel (DE)**
Erfinder: **Jansen, Rolf, Dr.
Falkenbergstrasse 14
D-3300 Braunschweig (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 216 731 B1

(72) Erfinder: **Kunze, Brigitte, Dr.**
**Hinter Aegidien 5**
**D-3300 Braunschweig (DE)**
Erfinder: **Schomburg, Dietmar, Dr.**
**Lübeckstrasse 50**
**D-3300 Braunschweig (DE)**
Erfinder: **Steinmetz, Heinrich**
**Schildweg 44**
**D-3200 Hildesheim-Sorsum (DE)**
Erfinder: **Trowitzsch-Kienast, Wolfram, Dr.**
**Im Dorfe 19 A**
**D-3300 Braunschweig (DE)**
Erfinder: **Wray, Victor, Dr.**
**Oeselweg 2**
**D-3300 Braunschweig (DE)**

(74) Vertreter: **Boeters, Hans Dietrich, Dr. et al**
**Boeters & Bauer, Patentanwälte Thomas-Wimmer-Ring 14**
**D-8000 München 22 (DE)**

**Beschreibung**

Die Erfindung betrifft neue makrozyklische Verbindungen, insbesondere das Sorangicin A, ein fermentatives Herstellungsverfahren unter Verwendung eines neuen Mikroorganismus der Species Sorangium cellulosum, den neuen Mikroorganismus selbst, pharmazeutische Präparate, welche die neuen Verbindungen enthalten, die therapeutische Verwendung der Verbindungen als Antibiotika und ihre Verwendung zur Herstellung von pharmazeutischen Präparaten.

Die Erfindung betrifft insbesondere makrozyklische Verbindungen der Formel

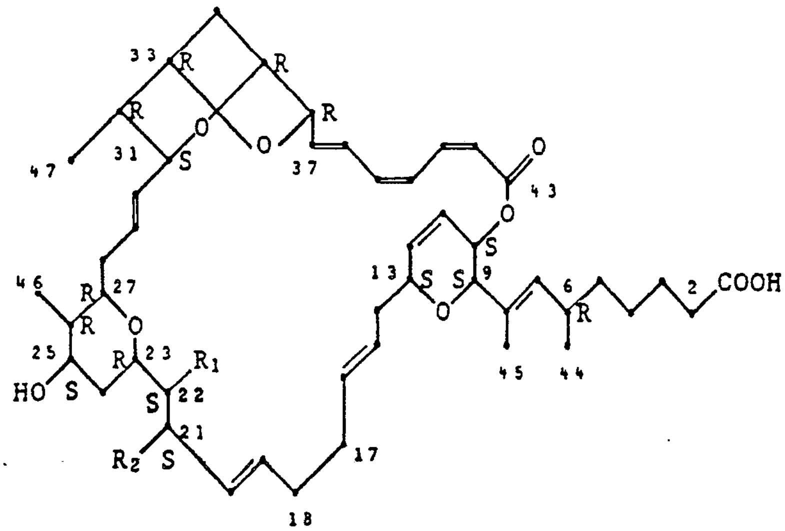

(I) ,

worin $R_1$ Wasserstoff oder Hydroxy und $R_2$ Hydroxy oder beta-Glucopyranosyloxy bedeuten, Solvate und Salze solcher Verbindungen, insbesondere pharmazeutisch verwendbare Salze.

Die Konfiguration der Substituenten an den chiralen C-Atomen wurde anhand einer Röntgenstrukturanalyse der kristallinen Verbindung der Formel I Sorangicin A ($R_1$ = OH, $R_2$ = OH) ermittelt und nach den Sequenzregeln der R,S-Nomenklatur von Cahn, Ingold, Prelog festgelegt. Wenn $R_1$ Hydroxy bedeutet, hat das C-22-Atom die S-Konfiguration.

Die Erfindung betrifft vor allem die fermentativ erhältliche Hauptkomponente der Formel I mit der Bezeichnung Sorangicin A ($R_1$ = OH, $R_2$ = OH, C—22:S), sowie die Nebenkomponenten Sorangicin B ($R_1$ = H, $R_2$ = OH), Sorangiosid A ($R_1$ = OH, $R_2$ = beta-Glucopyranosyloxy C—22:S), und Sorangiosid B ($R_1$ = H, $R_2$ = beta-Glucopyranosyloxy).

Ein weiterer bevorzugter Gegenstand der vorliegenden Erfindung ist der hinterlegte und im folgenden beschriebene Mikroorganismus der Species Sorangium cellulosum, welcher für das fermentative Herstellungsverfahren der Hauptkomponente Sorangicin A sowie der genannten Nebenkomponenten verwendet wird.

Die Verbindungen der Formel I können als Solvate vorliegen, z.B. kann Sorangicin A als kristallines Essigestersolvat isoliert werden.

Salze sind in erster Linie die pharmazeutisch verwendbaren oder nicht-toxischen Salze von Verbindungen der Formel I. Solche Salze sind in erster Linie geeignete Alkalimetall-, wie Natrium- oder Kalium-, oder Erdalkalimetallsalze, z.B. Magnesium- oder Calciumsalze, ferner Zinksalze, oder Ammoniumsalze, inkl. solche Salze, welche mit organischen Aminen, wie gegebenenfalls durch Hydroxy substituierten Mono-, Di- oder Trialkylaminen gebildet werden, z.B. Diäthylamin, Di(2-hydroxyäthyl)amin, Triäthylamin, N,N-Dimethyl-N-(2-hydroxyäthyl)-amin, Tri(2-hydroxyäthyl)amin oder N-Methyl-D-glucamin. Salze, die nicht pharmazeutisch verwendbar sind, z.B. schwerlösliche und/oder gut kristallisierende Salze können zur Isolierung und Reinigung verwendet werden.

Die Verbindungen der Formel I, insbesondere die fermentativ erhältliche Hauptkomponente Sorangicin A, sowie pharmazeutisch verwendbare Salze davon sind aktive, in der Human- und Veterinärmedizin therapeutisch verwendbare Antibiotika. Ihre Wirkung richtet sich gegen Kokken, Bakterien und Viren. Bekämpfbare Viren sind insbesondere solche, die zur Vermehrung reverse Transkriptase benötigen (Retroviren).

Beispielsweise werden *in vitro* im Agar-Verdünnungstest (H. M. Ericsson and S. C. Sherris, Acta Path. Microb. Scand. Section B, Suppl. No. 217, 1—90, 1971) MIC-Werte (Minimum Inhibitory Concentration) gefunden, welche für aerobe, grampositive und gramnegative Kokken, z.B. *Staphylococcus aureus, Neisseria gonorrhoeae, N. meningitidis, Streptococcus spp.* 0,01 bis 2 µg/ml betragen, und für *Haemophilus influenzae, Pseudomonas aeruginosa*, Enterobacterien, z.B. *Escherichia coli, Klebsiella pneumoniae, Proteus spp., Enterobacter cloacae, Serratia marcescens* oder Anaerobier, z.B. *Bacteroides fragilis* oder *Clostridium perfringens* im Bereich von ca. 0,01 bis 16 µg/ml liegen. Für Mykobakterien, z.B. *M. tuberculosis* und atypische Mykobacterien werden MIC zwischen 0,5 bis 8 µg/ml bestimmt.

Im *in vitro* Test mit polymorphkernigen menschlichen Leukozyten und *Staphylococcus aureus* Stamm Wood 46 ist Sorangicin A aktiv gegen intrazellulär vorkommende Bakterien, z.B. Staphylokokken; die Substanz weist eine Bakterien-abtötende Wirksamkeit im Inneren von Leukozyten auf.

Die gegen Retroviren gerichtete antivirale Wirkung der Verbindungen der Formel I wird durch *in vitro* und *in vivo* Tests nachgewiesen, insbesondere jedoch durch ein *in vitro* Testsystem, in welchem die Hemmung des Enzyms reverse Transkriptase durch besagte Verbindungen quantitativ bestimmt wird.

An Poly A-oligo (dT) wird mit der reversen Transkriptase radioaktiv markiertes TTP anhybridisiert. Die gebundene Radioaktivität wird gemessen und dient als Mass für die Aktivität der reversen Transkriptase. Zur Bestimmung der $ID_{50}$ der Verbindungen der Formel I werden diese in verschiedenen Konzentrationen in dem Testsystem eingesetzt.

Sorangicin A besitzt Aktivität gegen reverse Transkriptase von Moloney Murine Leucemia Virus; der $ID_{50}$ Wert [Dosis die die RT-Aktivität zu 50% hemmt; nach Wu et al., Proc. Natl. Acad. Sci, *69*, 3820 (1972)] für die RT MLV liegt bei 7 µg/ml.

Verbindungen der Formel I werden hergstellt, indem man den Stamm So ce 12 (NCIB 12134) der Species Sorangium cellulosum oder eine von diesem Stamm abgeleitete, die Verbindungen der Formel I' produzierende Mutante, in einer C- und N-Quelle und essentielle anorganische Salze enthaltenden Kultur bei ca. 15° bis 40°C und einem pH-Wert von ca. 4,0 bis 8,0 unter aeroben Bedingungen züchtet und, wenn erwünscht, die gebildeten Verbindungen der Formel I isoliert und/oder eine erhältliche Verbindung in ein Salz, und/oder ein erhältliches Salz in die freie Verbindung oder in ein anderes Salz überführt.

Der Mikroorganismus So ce 12 der Species *Sorangium cellulosum* wurde aus einer Bodenprobe aus der Umgebung von Xcaret, Halbinsel Yucatan, Mexico, isoliert und lässt sich als Stamm des Myxobacteriums *Sorangium cellulosum* klassifizieren.

Der Stamm So ce 12 ist am 1. August 1985 bei The National Collections of Industrial and Marine Bacteria Ltd. in Aberdeen, Schottland, Grossbritannien, unter der Nummer NCIB 12134 gemäss den Vorschriften des Budapester Vertrages über die internationale Anerkennung der Hinterlegung von Mikroorganismen für die Zwecke von Patentverfahren hinterlegt worden. Eine Lebensfähigkeitsbescheinigung ist von dieser Hinterlegungsstelle am 12. August 1985 ausgestellt worden.

Isolierung und Beschreibung von So ce 12

Die Bodenprobe wurde auf Filterpapier über Stan 21-Agar (0,1% $K_2HPO_4$, 0,1% $KNO_3$, 0,1% $MgSO_4 \cdot 7H_2O$, 0,01% $MnSO_4 \cdot 7H_2O$, 0,1% $CaCl_2 \cdot 2H_2O$, 0,02% $FeCl_3$, Standard Spurenelement-Lösung (bestehend z.B. aus je 20 mg/l $Na_2MoO_4 \cdot 2H_2O$, $Na_2B_4O_7 \cdot 10H_2O$, $MnSO_4 \cdot H_2O$ und $CuSO_4 \cdot H_2O$), 0.002% Hefeextrakt (Difco), 1% Agar, 25 mg/l Cycloheximid (Actidion) ausgelegt und drei Wochen lang bei 30° inkubiert.

Aufgetretene Schwarmkolonien mit Fruchtkörpern wurden erneut auf Filterpapier über Stan 21-Agar überimpft. Auftretende Amöben wurden durch Begasen mit einer fünfprozentigen, wässrigen Ammoniaklösung entfernt. Anschliessend wurden die Fruchtkörper in EBS-Lösung (0,5% Pepton aus Casein, tryptisch verdaut, Merck Darmstadt DE, 0,5% Proteosepepton, Difco, 0,1% Pepton aus Fleischextrakt, Merck, 0,1% Hefeextrakt, Difco, im Autoklaven sterilisiert) über Nacht mit Antibiotika Lösung AB—1 (20 mg Chloramphenicol, 30 mg Streptomycinsulfat, 25 mg Tetracyclinhydrochlorid, 20 mg Cephalotin, aufgelöst in 50 ml Wasser und sterilfiltriert), bei 4°C aufbewahrt und danach auf vy/2-Agar (0,5% Bäckerhefe bezogen auf Frischgewicht, 0,1% $CaCl_2 \cdot 2H_2O$, 1,5% Agar, pH 7,2) ausgestrichen. Auswachsende, reine Schwarmkolonien wurden auf demselben Medium weiterkultiviert sowie auf Filterpapier über Stan-21-Agar auf Identität mit dem Ausgangsisolat überprüft.

Die vegetativen Zellen sind zylindrische Stäbchen mit runden Enden, meist ca. 1 µm breit und 3—6 µm lang. Im Phasenkontrastmikroskop erscheinen sie dunkel. Sie bewegen sich gleitend fort. Auf manchen Nährböden bildet der Organismus massenhaft Fruchtkörper, so z.B. auf Filterpapier über Mineralsalzagar (Stan 21 Agar). Die Fruchtkörper ähneln rostfarbenen Polstern und bestehen aus einer mehr oder weniger grossen Zahl von Sporangiolen, kugeligen oder durch gegenseitige Abplattung polyedrischen Gebilden mit einer festen Wand von 20 bis 30 mµ Durchmesser. In den Sporangiolen befinden sich Myxosporen, stäbchenförmige Dauerzellen von ähnlicher Gestalt und Grösse wie die vegetativen Zellen, jedoch leicht lichtbrechend.

Der Stamm So ce 12 kann spontan Mutanten bilden (natürliche Mutanten) oder es lassen sich künstliche Mutanten herstellen, die ebenso wie der natürliche Stamm antibiotisch wirksame Verbindungen der Formel I produzieren.

Solche Mutanten kann man chemisch, z.B. durch Behandlung mit gewissen Guanidinderivaten, z.B. N-Methyl-N'-nitro-N-nitrosoguanidin oder mit Alkalinitrit, z.B. Natriumnitrit, oder physikalisch, z.B. durch energiereiche Strahlung, z.B. Ultraviolett-, Röntgen- oder radioaktive Strahlung, erzeugen.

Die zur Züchtung verwendbare Kultur muss eine Kohlenstoff- und Stickstoffquelle sowie essentielle anorganische Salze enthalten. Als Kohlenstoffquelle sind beispielsweise zu nennen: assimilierbare Kohlehydrate, z.B. D-Glucose, D-Xylose, L-Arabinose, D-Fructose, Maltose, Maltotriose, Stärke. Als Stickstoffquellen seien genannt: Aminosäuren, Peptide und Proteine sowie deren Abbauprodukte wie Pepton oder Trypton, ferner Fleischextrakte, Getreidemehl, z.B. von Mais oder Weizen, Bohnen, insbesondere Sojabohnen, Samen, beispielsweise der Baumwollpflanze, Destillationsrückstände der Alkoholherstellung, Hefeextrakte usw., aber auch Ammoniumsalze und Nitrate. Als essenzielle anorganische Salze kann die

Nährlösung beispielsweise Chloride, Carbonate, Sulfate, Phosphate von Alkali- oder Erdalkalimetallen z.B. Natrium, Kalium, Magnesium, Calcium, Eisen, Zink, Mangan, Molybdän und Kupfer enthalten. Die Züchtung erfolgt bevorzugt in Flüssigkulturen, insbesondere wässrigen Kulturen.

Als flüssiges Kulturmedium eignet sich insbesondere das Medium MD1 (Pepton aus Casein, tryptisch verdaut, Merck, 0,3%; $CaCl_2 \cdot 2H_2O$ 0,05%; $MgSO_4 \cdot 7H_2O$ 0,2%), das durch eine Kohlenhydratquelle ergänzt ist, z.B. Glucose, Maltose, Maltotriose, Stärke, Cellulose, jeweils 0,1%.

Ein weiteres brauchbares flüssiges Kulturmedium enthält Pepton aus Casein, 0,1%; $CaCl_2 \cdot 2H_2O$, 0,05%; $MgSO_4 \cdot 7H_2O$, 0,2%; Corn steep Pulver oder Maiskleber 0,4%. Der Stamm ist auch in einem definierten Medium kultivierbar, welches aus $MgSO_4 \cdot 7H_2O$ 0,15%; $FeCl_3 \cdot 6H_2O$ 8 mg/l; $KNO_3$ 0,2%; $K_2HPO_4$ 0,025%; Glucose·$H_2O$ 0,5%; $CaCl_2 \cdot 2H_2O$ 0,15% besteht. Zusatz von 0,01—0,05% Pepton führt zu homogenem Wachstum, einer besseren Zellausbeute und zu höherer Antibiotika-Bildung.

Die Züchtung kann ansatzweise, z.B. durch ein- oder mehrmalige Zugabe von Nährlösung, oder kontinuierlich durch dauernde Zugabe von Nährlösung erfolgen.

Vorzugsweise züchtet man in mehreren Stufen, indem man eine Vorkultur, z.B. in einem der genannten Kulturmedien, herstellt (Inoculum), welche man anschliessend nach ca. ein- bis zweitägiger Fermentation in die eigentliche Hauptkultur, vorzugsweise in einem Verdünnungsverhältnis 1:10, überimpft.

Diese Vorkultur erhält man beispielsweise aus einer Reihe von Vorkulturen. Die erste Kultur dieser Reihe erhält man durch 14-tägiges Wachstum des betreffenden Stammes auf festem oder flüssigem Nährboden, z.B. Agar + MD1 + 0,1% Stärke. Die Bakterienmasse wird anschliessend in eine Nährlösung überimpft und mehrere Tage, z.B. 4—5 Tage, inkubiert. Diese Nährlösung kann, falls gewünscht, in eine weitere Nährlösung, z.B. MD1 + 0.1% Stärke, oder in zeitlicher Abfolge von ca. 4—5 Tagen in mehrere Nährlösungen, z.B. in einem Verdünnungsverhältnis von ca. 3% (v/v), überimpft und unter den genannten Bedingungen inkubiert werden.

Man kann den Fermentationsverlauf durch Probenentnahme analytisch während der Fermentation verfolgen, z.B. durch Messung des pH-Wertes der Kultur, welcher während der Fermentation von ca. 7,2 auf etwa 6,0—6,5 absinkt und dann bis ca. 7,5—8,0 ansteigt, oder der optischen Dichte, welche ein Mass für das Wachstum des jeweiligen Stammes ist, sowie gravimetrisch anhand des Trockengewichts der gebildeten Zellmasse, durch Dünnschichtchromatographie oder durch Bestimmung der antibiotischen Aktivität der im Kulturfiltrat enthaltenen Komponenten.

Die Isolierung der Verbindungen der Formel I, insbesondere der Hauptkomponente Sorangicin A, aus der Kulturbrühe erfolgt nach an sich bekannten Methoden unter Berücksichtigung der chemischen, physikalischen und biologischen Eigenschaften der Substanzen. Zum Bestimmen der Antibiotika-Konzentration in den einzelnen Isolierungsstufen — wie auch im Kulturmedium — kann die Dünnschichtchromatographie, z.B. auf Silicagel (z.B. mit Methylenchlorid Methanol), und/oder die Aktivität gegen verschiedene Mikroorganismen, z.B. Staphylococcus aureus, und/oder die Hemmung der reversen Transkriptase, verwendet werden.

Für die Isolierung der Verbindungen der Formel I aus der rohen Fermentationsbrühe eignen sich zwei Methoden:

a) Mehrstündiges Rühren der Fermentationsbrühe mit makroporösen, nicht-ionischen Adsorberharzen, z.B. synthetischen Harzen mit aromatischem Grundgerüst, beispielsweise Harzen auf Polystyrolbasis, z.B. Styrol-Divinylbenzol-Copolymere. Solche Harze lassen sich durch verschiedene gebräuchliche statistische Kenngrössen, z.B. Porenvolumen (pore volume), spezifische Oberfläche (specific surface area), durchschnittliche Porendurchmesser (average pore diameter), häufigster Porendurchmesser (most frequent pore diameter), Porengrössenverteilung (pore size distribution), Körngrössenverteilung (bead size distribution) und dergleichen charakterisieren.

Geeignete Adsorberharze haben ein Porenvolumen von ca. 0,5 bis ca. 4,5 ml/g, eine spezifische Oberfläche von ca. 100—1000 $m^2/g$ und einen durchschnittlichen Porendurchmesser von ca. 4 bis ca. 130 nm und sind beispielsweise unter den Handelsnamen AMBERLITE® XAD—1, XAD—2, XAD—4, XAD—1180 und ER—180 der Fa. Rohm & Haas, DIAION® HP—10, HP—20, HP—21, HP—30, EP—40, HP—50 der Fa. Mitsubishi, DUOLITE® S—861, S—862, S—863 und ES 866 der Fa. Dia-Prosim, IMAC® Syn 46 und Syn 72 der Firma Akzo Chemie, KASTEL S—111, S—112, S—114 der Fa. Montedison, LEWATIT® OC.1031 der Fa. Bayer und RELITE® ADS der Fa. Resindion, erhältlich.

Nach Abtrennung der Fermentationsbrühe vom Adsorberharz, z.B. durch Sieben, wird dieses mit Wasser gewaschen und anschliessend mit einem Gemisch von Wasser mit steigenden Anteilen eines organischen Lösungsmittels, welches gegenüber dem verwendeten Adsorberharz inert ist, z.B. einem Wasser-Methanol Gemisch, eluiert, wobei mit 80—100 %igem Methanol die Hauptmenge der Verbindungen der Formel I eluiert wird. Die Eluate werden im Vakuum eingeengt und, wie im folgenden unter Variante b) beschrieben, durch HPLC aufgetrennt.

b) Die Kulturbrühe wird von der Zellmasse auf übliche Weise, z.B. durch Filtration oder Zentrifugation, abgetrennt und das Kulturfiltrat mit einem mit Wasser nur wenig oder nicht mischbaren organischen Lösungsmittel, z.B. Methylenchlorid, Chloroform oder bevorzugt Essigsäureäthylester, extrahiert. Nach Eindampfen der organischen Phase im Vakuum erhält man einen Rohextrakt. Bei Verteilung des Rohextrakts im Zweiphasensystem bestehend aus zwei nicht miteinander mischbaren organischen Lösungsmitteln, z.B. Methanol-Heptan, reichern sich die Fermentationsprodukte, insbesondere die Antibiotika der Formel I', in der polaren Phase an. Nach Abdampfen des polaren Lösungsmittels wird der

Rückstand in verdünnter, wässriger Ammoniaklösung gelöst und mit einem mit Wasser nicht mischbaren organischen Lösungsmittel, z.B. Diäthyläther, gewaschen. Im Vakuum wird das flüchtige Ammoniak entfernt und die wässrige Phase nach Ansäuern mit einer organischen Säure, z.B. Ameisen- oder Essigsäure, mit einem der genannten mit Wasser nur wenig oder nicht mischbaren Lösungsmittel, z.B. Methylenchlorid, extrahiert. Anschliessend werden die erhältlichen Antibiotika durch Umkehrphasenchromatographie (Reversed Phase Chromatography) gereinigt. Man erhält mehrere Fraktionen, in denen Sorangiosid A und B im Gemisch oder in reiner Form und Sorangicin A und B im Gemisch oder in reiner Form enthalten sein können. Die Einzelkomponenten Sorangiosid A und Sorangiosid B sowie Sorangicin B erhält man durch Trennung der entsprechenden Fraktionen mit HPLC, während die Hauptkomponente Sorangicin A aus der betreffenden Fraktion kristallin erhalten werden kann.

Die Hauptkomponente Sorangicin A kann auch nach anderen Varianten dieser an sich bekannten Trennmethoden, z.B. durch Verwendung anderer Lösungsmittel bzw. Lösungsmittelgemische oder Verwendung anderer chromatographischer Methoden, erhalten werden.

Salze von Verbindungen der Formel I können in an sich bekannter Weise hergestellt werden. So kann man Salze von Verbindungen der Formel I z.B. durch Behandeln mit Metallverbindungen, wie Alkalimetallsalzen von geeigneten organischen Carbonsäuren, z.B. dem Natriumsalz der α-Aethylcapronsäure, oder mit einem anorganischen Alkali- oder Erdalkalimetallsalz, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet.

Salze können in üblicher Weise in die freien Verbindungen übergeführt werden. Metall- und Ammoniumsalze z.B. durch Behandeln mit geeigneten Säuren.

Stereoisomerengemische, insbesondere Diastereomerengemische, können in an sich bekannter Weise, z.B. durch fraktionierte Kristallisation, Chromatographie, in die einzelnen Isomere aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, oder durch spezifische Mikroorganismen aufgetrennt werden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die abschliessenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Die pharmakologisch verwendbaren Verbindungen der vorliegenden Erfindung können z.B. zur Herstellung von pharmazeutischen Präparaten Verwendet werden, welche eine wirksame Menge des Wirkstoffs vorzugsweise im Gemisch mit einer signifikanten Menge von anorganischen oder organischen, festen oder flüssigen, pharmazeutisch verwendbaren Trägerstoffen enthalten. Pharmazeutische Präparate, ihre Herstellung und Verwendung, sind ebenfalls Gegenstand der vorliegenden Erfindung.

Die erfindungsgemässen pharmazeutischen Präparaten eignen sich zur parenteralen, z.B. i.v., i.m., gegebenenfalls auch oralen oder topischen Verabreichung.

Beispielsweise verwendet man die Wirkstoffe der Formel I der vorliegenden Erfindung in Form von injizierbaren, z.B. intravenös, verabreichbaren Präparaten oder Infusionslösungen. Solche Lösungen sind vorzugsweise sterilisierte isotonische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebrauch hergestellt werden können. Pharmazeutische Präparate zur oralen Anwendung sind gegebenenfalls sterilisiert und können Hilfsstoffe, z.B. Konservier-, Stabilisier-, Netz- und/oder Emulgiermittel, Löslichkeits- vermittler, Salze zur Regulierung des osmotischen Druckes, Resorptionsverstärker und/oder Puffer enthalten. Die vorliegenden pharmazeutischen Präparate, die, wenn erwünscht, weitere pharmakologisch wertvolle Stoffe, z.B. andere Wirkstoffe, enthalten können, enthalten etwa 0,1% bis 100%, insbesondere etwa 1% bis zu 100%, des Wirkstoffes.

Die pharmazeutischen Präparate werden in an sich bekannter Weise, z.B. mittels konventioneller in Pharmakopöen beschriebener Lösungs- oder Lyophilisierungsverfahren, hergestellt.

Verwendung

Verbindungen der Formel I, deren Solvate oder pharmazeutisch verwendbaren Salze können als Antibiotika in Form von pharmazeutischen Präparaten zur therapeutischen Behandlung des menschlichen oder tierischen Körpers angewendet werden, z.B. als antibakterielle Antibiotika zur Behandlung von Infektionen, die durch grampositive oder gramnegative Bakterien verursacht werden, wie z.B. durch *Neisseria gonorrhoeae* oder *meningitidis, Staphylococcus aureus* oder *epidermidis*, Streptokokken, inkl. Enterokokken, *Haemophilus influenzae, Pseudomonas aeruginosa*, oder anaerobe Erreger, wie z.B. *Bacteroides fragilis* oder *Clostridia* spp., Infektionen, die durch Bakterien verursacht werden, die in Zellen, z.B. Leukozyten, überleben, wie z.B. Staphylokokken oder Listerien, Tuberkulose oder Infektionen, die von atypischen Mykobakterien, wie z.B. *Mycobacterium intracellulare avium*, verursacht werden.

Die erfindungsgemässen Verbindungen können auch als antivirale Antibiotika eingesetzt werden, insbesondere zur Behandlung von Infektionen, die durch Viren mit reverser Transkriptase Aktivität, wie z.B. Retroviren (u.a. AIDS verursachendes HTLV III), oder Hepatitis Viren, etc. verursacht werden. Neben

Infektionen mit HTLV III (auch als LAV I oder HIV bezeichnet) kommen noch solche mit HTLV I, HTLV II und HTLV IV (auch als LAV II bezeichnet) in Frage.

Je nach Art, Schwere und Dauer der Infektion, dem Zustand des Patienten und der Verabreichungsweise verwendet man tägliche Dosen von etwa 0,3 g bis zu etwa 10,0 g s.c., i.m., i.v., p.o. oder per inhalationem zur Behandlung von Personen oder Warmblütern von etwa 70 kg Körpergewicht.

Weitere Verwendungsmöglichkeiten bestehen im Pflanzenschutz, z.B. bei der Bekämpfung von Bakterienkrankheiten von Gewächshauskulturen, bei der Pflege von infizierten Bäumen etc. Für diese Verwendung eignen sich z.B. Aerosole.

Die nachfolgenden Beispiele illustrieren die experimentelle Realisierung und generelle Operabilität der vorliegenden Erfindung. Temperaturen sind in Graden Celsius angegeben.

## Beispiel 1

a) Herstellung des Inoculums der Vorkultur

Von einer ca. 14 Tage alten Agarplatte, enthaltend das Medium MD1 (0.3% Pepton aus Casein, tryptisch verdaut, Fa. Merck DE, 0,05% $CaCl_2 \cdot 7H_2O$, 0,2% $MgSO_4 \cdot 7H_2O$) und 0,1% Stärke, werden Bakterien des Stammes So ce 12 in 100 ml flüssiges MD1-Medium mit 0,1% Stärke überimpft und auf einem Schütteltisch bei 30° und 160 Upm (Umdrehungen pro Minute) inkubiert. Nach ca. 5 Tagen Inkubationszeit wird die angewachsene Kultur in einem Verdünnungsverhältnis von 3% (v/v) in neues Nährmedium MD1 überimpft. Nach jeweils 4 Tagen Inkubationszeit kann noch sooft wie erforderlich in einem Verdünnungsverhältnis von 3% (v/v) in neues Nährmedium MD1 überimpft werden.

Die letzte Vorkultur dieser Reihe dient in einem Verdünnungsverhältnis von 10% (v/v) als Inoculum für 4 l einer weiteren Vorkultur mit MD1-Nährlösung. Diese Vorkultur wird bei 30° inkubiert, etwas gerührt und unter schwacher Luftzufuhr begast. Nach 2 Tagen Inkubationszeit wird zur Herstellung der eigentlichen Vorkultur ein 70 l Fermenter mit diesem Inoculum angeimpft.

b) Herstellung der Vorkultur

70 l Fermenter, Fa. Giovanola, Monthey VS, Schweiz, Blattrührsystem, Medium MD1 (1,0 m) mit 0,1% Stärke, T = 30°. Rührgeschwindigkeit 320 Upm. Luftzufuhr 0,3 $Nm^3/h$. Der prozentuale Sauerstoffpartialdruck sinkt im Verlauf der Fermentation ab und erreicht nach 2 Tagen einen Wert von 10—20%. $OD_{623}$: ca. 2. Ein 700 l Fermenter wird mit dieser Vorkultur unter sterilen Bedingungen angeimpft.

c) Hauptkultur

700 l Fermenter mit Blattrührsystem der unter b) genannten Firma. Medium: 0,15% Magnesiumsulfatheptahydrat, 8 mg/l Eisen(III)-chloridhexahydrat, 0,05% Pepton, 0,2% $KNO_3$, 0,025% $K_2HPO_4$, 0,5% Glucose, 0,15% Calciumchloriddihydrat, pH 7,3. Rührgeschwindigkeit am Anfang: 150 Upm. Rührgeschwindigkeit nach 23 Stunden: 100 Upm. Prozentualer Sauerstoffpartialdruck am Ende der Fermentation: 10%. Ende der Fermentation nach 40 Stunden. Am Ende der Fermentation zeigt der Ueberstand einen Hemmhofdurchmesser gegen *Staphylococcus aureus* von ca. 14—15 mm. Die Zellmasse wird von der Kulturbrühe durch Zentrifugation getrennt. Die antibiotisch wirksamen Komponenten sind im Kulturfiltrat enthalten. Sämtliche Verfahrensschritte werden unter sterilen Bedingungen durchgeführt.

d) Aufarbeitung des Kulturfiltrats

Das Kulturfiltrat wird mit Essigsäureäthylester extrahiert. Nach Eindampfen des organischen Lösungsmittels erhält man einen Rohextrakt. Bei Verteilung im Zweiphasensystem Heptan-Methanol reichern sich die Verbindungen der Formel I in der methanolischen Phase an. Nach Eindampfen wird der Rückstand in 12 %-iger wässriger Ammoniaklösung gelöst, die Lösung mehrfach mit Aether gewaschen und im Vakuum vom Ammoniak befreit. Nach dem Ansäuern der wässrigen Phase mit Ameisensäure wird mit Methylenchlorid extrahiert. Nach dem Eindampfen des Extrakts wird mit Umkehrphasenchromatographie getrennt. Säule: Labochrom® PGC-Säule 674 × 37 mm (Fa. Labomatic), gepackt mit LiChroprep® RP—18 (25—40 µm) (Merck), Laufmittel = Methanol/Puffer 68:32, Puffer = 0.5% Ameisensäure in Wasser, mit Triethylamin auf pH 7 eingestellt, Fliessgeschwindigkeit: 30 ml/min, Detektion: UV-Absorption bei 313 nm. Man erhält folgende Fraktionen:

Fraktion 1: Peak bei $t_R$ = 31 min: Sorangiosid A + B
Fraktion 2: Peak bei $t_R$ = 40 min: Sorangiosid B
Fraktion 3: Peak bei $t_R$ = 50 min: Sorangicin A
Fraktion 4: Peak bei $t_R$ = 69 min: Sorangicin B

Nach Entfernen des organischen Lösungsmittels werden die in den einzelnen Fraktionen befindlichen Komponenten aus der wässrigen, gepufferten Phase mit Dichlormethan oder Essigester extrahiert.

Fraktion 1: Das Gemisch aus Sorangiosid A und B wird durch HPLC getrennt: Säule 250 × 16 mm (Knauer), gepackt mit LiChrosorb Si 100 (10 µ) (Merck), Detektion durch UV-Absorption bei 313 nm, Laufmittel = Dichlormethan/Heptan/i-Propanol/konz. Puffer 52:4:7:1, konz. Puffer aus Methanol/Ameisensäure 1:2 mit Triethylamin neutralisiert, Fliessgeschwindigkeit = 18 ml/min. Es wird Sorangiosid B mit einer Retentionszeit $t_R$ = 9.4 min und Sorangiosid A mit einer Retentionszeit $t_R$ = 13.8 min erhalten.

Sorangiosid A

HPLC: Säule 250 × 16 mm (Knauer), gepackt mit Nucleosil® 7 $C_6H_5$ (Macherey-Nagel), Laufmittel = Methanol/Wasser 65/35 + 0.5% Ameisensäure, Fliessgeschwindigkeit = 10 ml/min, Detektion: UV-Absorption bei 313 nm: $t_R$ = 26.4 min.

Dünnschichtchromatographie (Kieselgel Si 60 F 254 (Merck), Laufmittel Methylenchlorid/Methanol 8:2): $R_f$ = 0.44.

$[\alpha]_D^{22}$ = +42.0 (c = 0.9 in Methanol).

UV (Methanol): $\lambda_{max}$ (1gε) = 301 (4.34).

$^{13}C$—NMR s. Tabelle.

Fraktion 2: Die Sorangiosid B-haltige Fraktion wird durch HPLC nachgereinigt:Säule 250 × 16 mm (Knauer), gepackt mit Nucleosil 7 $C_6H_5$ (Macherey-Nagel), Detektion: UV-Absorption bei 313 nm: Laufmittel = Methanol/Wasser 70:30 + 0,5% Ameisensäure, Fliessgeschwindigkeit = 14 ml/min: $t_R$ = 10,2 min.

Sorangiosid B

Dünnschichtchromatographie (Kieselgel 60 F 254 (Merck), Laufmittel Methylenchlorid/Methanol 8:2): $R_f$ = 0,62.

$[\alpha]_D^{22}$ = +5.1 (c = 1.8 in Methanol).

UV (Methanol): $\lambda_{max}$ (1gε) = 301 (4.41).

$^{13}C$—NMR s. Tabelle.

Fraktion 3: Aus Essigester kristallisiert.

Sorangicin A

Smp.: 105—107°.

$[\alpha]_D^{22}$ = +60,9 (c = 0.7 in Methanol).

UV (MeOH): $\lambda_{max}$ (1gε) = 301 (4.33).

$^{13}C$—NMR s Tabelle.

FAB—MS [neg. Ionen, Xenon bei 9 keV (Iontect), Beschleunigung — 8 kV, Nachbeschleunigung 11 KV]: m/e = 805 $(M—H)^-$, 897 $(M—H + Glycerin)^-$.

EI—MS (70 eV, 245°): m/e % = 806 ($M^+$, 0,3), 789 (0,6), 788 (1,5), 770 (0,8), 373 (2), 303 (2,5), 301 (3,5), 249 (44), 231 (5), 197 (15), 149 (20), 135 (21), 133 (22), 121 (35), 109 (40), 107 (43), 105 (100).

Die Verbindung liegt als Essigestersolvat vor. Sie lässt sich auch aus Aceton umkristallisieren.

Analyse:

| | | | | |
|---|---|---|---|---|
| $C_{47}H_{66}O_{11} \times C_4H_8O_2$ | Ber. | C 68,43 | H 8,33 | O 23.23 |
| Gef. | | C 68,11 | H 8,35 | O 23.61 |

Fraktion 4: Nachreinigung durch HPLC: Säule 250 × 16 mm (Knauer), gepackt mit Nucleosil 7 $C_6H_5$ (Macherey-Nagel), Detektion UV-Absorption bei 313 nm. Laufmittel = Methanol/Wasser 75:25 + 0,5% Ameisensäure; Fliessgeschwindigkeit = 14 ml/min, Retentionszeit: $t_R$ = 11.7 min.

Sorangicin B

$[\alpha]_D^{22}$ = +49,1 (c = 1.6 in Methanol).

UV (Methanol): $\lambda_{max}$ (1gε) = 301 (4,41).

$^{13}C$—NMR s. Tabelle.

FAB—MS [neg. Ionen, Xenon bei 9 keV (Iontect), Beschleunigung −8 kV, Nachbeschleunigung 11 kV]: m/z = 789 $(M—H)^-$, 881 $(M—H + Glycerin)^-$.

EI—MS (70 eV, 270°): m/z (%) = 790 ($M^+$, 1), 773 (3), 772 (5), 755 (2), 754 (3), 643 (1), 250 (20), 249 (100), 197 (22), 191 (11), 181 (12), 169 (10), 163 (11), 161 (11), 159 (11), 149 (22), 133 (22), 123 (23), 121 (29), 107 (31), 105 (61).

Hochauflösung

$C_{47}H_{66}O_{10}$ Ber. 790,4656 Gef. 790,4618.

Tabelle
$^{13}$C-NMR Daten der Sorangicine und Sorangioside in $CD_3OD$

| No. | Sorangiosid[d)] | | Sorangicin | |
|---|---|---|---|---|
| C-Atom | A | B | A | B |
| 1 | 178.01s | 177.69s | 177.85s | 177.72s |
| 2 | 35.38t | 35.14t | 35.28t | 35.20t |
| 3 | 26.27t | 26.27t | 26.29t | 26.54t |
| 4 | 28.20t | 28.18t | 28.20t | 28.21t |
| 5 | 38.52t | 38.34t | 38.51t | 38.24t |
| 6 | 32.90d | 32.98d | 32.96d | 33.02d |
| 7 | a | a | a | a |
| 8 | 131.26s | 131.68s | 131.24s | 131.48s |
| 9 | 74.44d | 74.72d | 74.42d | 74.45d |
| 10 | 66.95d | 67.03d | 66.90d | 66.91d |
| 11 | 123.91d | 123.70d | 123.79d | 123.68d |
| 12 | 136.94d | 136.89d | 136.85d | 136.86d |
| 13 | 75.37d | 74.72d | 75.33d | 75.07d |
| 14 | b | b | b | b |
| 15 | a | a | a | a |
| 16 | a | a | a | a |
| 17 | b | b | b | b |
| 18 | b | b | b | b |
| 19 | 136.41d | 134.42d | 134.36d | 137.37d |
| 20 | 127.23d | 132.41d | 130.16d | 132.88d |
| 21 | 83.21d | 80.45d | 74.36d | c |
| 22 | 76.04d | 43.05t | 77.77d | 45.08t |
| 23 | 74.05d | 71.10d | 74.87d | c |
| 24 | 31.40t | 35.41t | 30.86t | 35.40t |
| 25 | 70.98d | 70.82d | 71.07d | 71.11d |
| 26 | 38.03d | 37.81d | 38.51d | 38.42d |
| 27 | 74.44d | 74.18d | 75.05d | 74.71d |
| 28 | b | b | b | b |
| 29 | a | a | a | a |
| 30 | a | a | a | a |
| 31 | 81.15d | 81.06d | 81.17d | 81.09d |
| 32 | 42.11d | 42.02d | 42.15d | 42.10d |
| 33 | 81.06d | 80.94d | 81.03d | 80.95d |
| 34 | 39.80t | 39.93t | 39.85t | 39.92t |
| 35 | 77.53d | 77.12d | 77.59d | 77.35d |
| 36 | 82.03d | 81.77d | 82.26d | 82.13d |
| 37 | 134.91d | 134.77d | 134.88d | 134.66d |
| 38 | 127.76d | 127.22d | 127.84d | 127.55d |
| 39 | 137.57d | 137.70d | 137.57d | 137.72d |
| 40 | 127.00d | 126.82d | 126.98d | 126.89d |
| 41 | 139.14d | 139.39d | 139.05d | 139.21d |
| 42 | 119.72d | 119.44d | 119.69d | 119.49d |
| 43 | 167.67s | 167.33s | 167.68s | 167.51s |
| 44 | 21.73q | 21.54q | 21.67q | 21.54q |
| 45 | 14.30q | 14.24q | 14.30q | 14.23q |
| 46 | 10.62q | 10.62q | 10.89q | 10.82q |
| 47 | 15.36q | 15.32q | 15.36q | 15.33q |

Fortsetzung Tabelle 2
Fussnoten:

a) Sorangiosid A: 134.28d, 133.77d, 133.06d, 132.41d, 128.31d;
Sorangiosid B: 135.06d, 133.76d, 133.11d, 132.41d, 128.40d;
Sorangicin A: 134.15d, 133.61d, 133.00d, 132.78d, 128.34d;
Sorangicin B: 134.27d, 133.53d, 133.03d, 132.70d, 128.40d;

b) Sorangiosid A: 37.06t, 35.38t, 34.37t, 33.61t;
Sorangiosid B: 37.01t, 35.41t, 33.85t, 33.64t;
Sorangicin A: 37.12t, 35.45t, 33,97t, 33.35t;
Sorangicin B: 37.13t, 35.40t, 33.49t, 33.49t;

c) 72.13d, 71.72d;

d) Glucosyl-Signale: C—1′ C—2′ C—3′ C—4′ C—5′ C—6′
Sorangiosid A: 102.81d, 75.19d, 78.13d, 71.64d, 77.83d, 62.80t
Sorangiosid B: 102.62d, 75.27d, 78.21d, 71.61d, 77.73d, 62.83t

Beispiel 2

Trockenampullen oder Vialen enthaltend 0.5 g Sorangicin A als Wirkstoff kann man folgendermassen herstellen:

Zusammensetzung: (für 1 Ampulle oder Viale)
Wirkstoff 0,5 g
Mannit 0,05 g

Eine sterile wässrige Lösung bestehend aus Wirkstoff und Mannit wird unter aseptischen Bedingungen in 5 ml-Ampullen oder 5 ml-Vialen eingefüllt, worauf diese verschlossen und geprüft werden.

**Patentansprüche**

1. Verbindungen der Formel

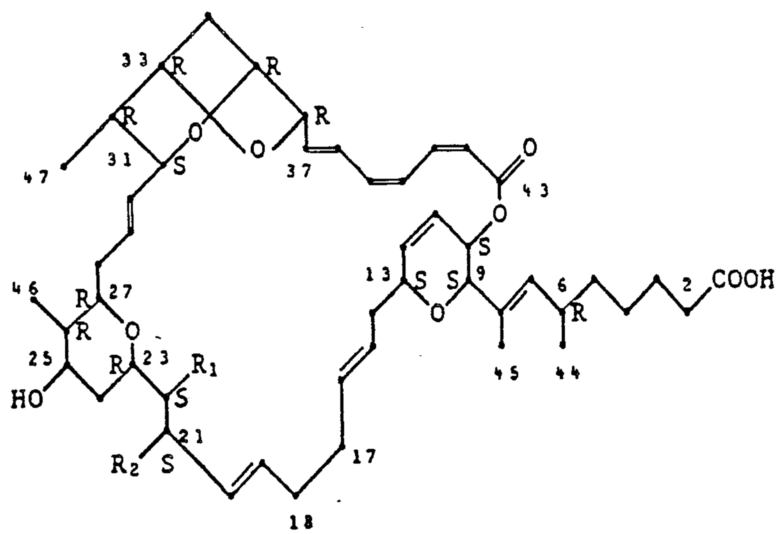

(I) ,

worin $R_1$ Wasserstoff oder Hydroxy und $R_2$ Hydroxy oder beta-Glucopyranosyloxy bedeuten, und wenn $R_1$ Hydroxy bedeutet, das C—22-Atom die S-Konfiguration hat, Solvate und Salze dieser Verbindungen.

2. Die Verbindung der Formel I, worin $R_1$ Hydroxy und $R_2$ Hydroxy ist (Sorangicin A) und das C—22-Atom die S-Konfiguration hat.

3. Das Essigestersolvat der Verbindung gemäss Anspruch 2.

4. Die Verbindungen der Formel I, worin $R_1$ Wasserstoff, $R_2$ Hydroxy (Sorangicin B), $R_1$ Hydroxy und $R_2$ beta-Glucopyranosyloxy (Sorangiosid A C—22:S) oder $R_1$ Wasserstoff und $R_2$ beta-Glucopyranosyloxy (Sorangiosid B) sind.

5. Verfahren zur Herstellung von Verbindungen der Formel I, Solvate oder Salzen davon gemäss Anspruch 1, dadurch gekennzeichnet, dass man den Stamm So ce 12 (NCIB 12134) der Species Sorangium cellulosum oder eine von diesem Stamm abgeleitete, die Verbindungen der Formel I produzierende Mutante in einer C- und N-Quellen und essentielle anorganische Salze enthaltenden Kultur bei ca. 20° bis 40°C und einem pH-Wert von ca. 4,0 bis 8,0 unter aeroben Bedingungen züchtet und, wenn erwünscht, die erhältlichen Verbindungen der Formel I gegebenenfalls als Hydrat isoliert, und eine erhältliche Verbindung in ein Salz, und/oder ein erhältliches Salz in die freie Verbindung oder in ein anderes Salz überführt.

6. Der Stamm So ce 12 (NCIB 12134) der Species Sorangium cellulosum oder eine von diesem Stamm abgeleitete Mutante, welche eine Verbindung der Formel I produziert.

7. Die nach dem Verfahren gemäss Anspruch 5 erhältlichen antibiotisch wirksamen Verbindungen.

8. Pharmazeutische Präparate enthaltend eine Verbindung der Formel I, ein Solvat oder ein pharmazeutisch verwendbares Salz davon gemäss Anspruch 1.

9. Verbindungen der Formel I, Hydrate oder pharmazeutisch verwendbare Salze davon gemäss Anspruch 1 zur Anwendung in einem Verfahren zur therapeutischen Behandlung des menschlichen oder tierischen Körpers.

10. Verbindungen der Formel I, Solvate oder pharmazeutisch verwendbare Salze davon gemäss Anspruch 1 zur Anwendung im Pflanzenschutz.

11. Verwendung einer Verbindung der Formel I, eines Solvates oder pharmazeutisch verwendbaren Salzes davon gemäss Anspruch 1 zur Herstellung eines pharmazeutischen Präparates.

12. Verwendung einer Verbindung der Formel I, eines Solvates oder eines pharmazeutisch verwendbaren Salzes davon gemäss Anspruch 1 zur Herstellung eines antibakteriellen Präparates gemäss Anspruch 11.

13. Verwendung einer Verbindung der Formel I, eines Solvates oder pharmazeutisch verwendbaren Salzes davon gemäss Anspruch 1 zur Herstellung eines antiviralen Präparates gemäss Anspruch 11.

14. Verwendung einer Verbindung der Formel I, eines Solvates oder Salzes davon gemäss Anspruch 1 zur Herstellung eines Pflanzenschutzpräparaten.

**Revendications**

1. Composés répondant à la formule I:

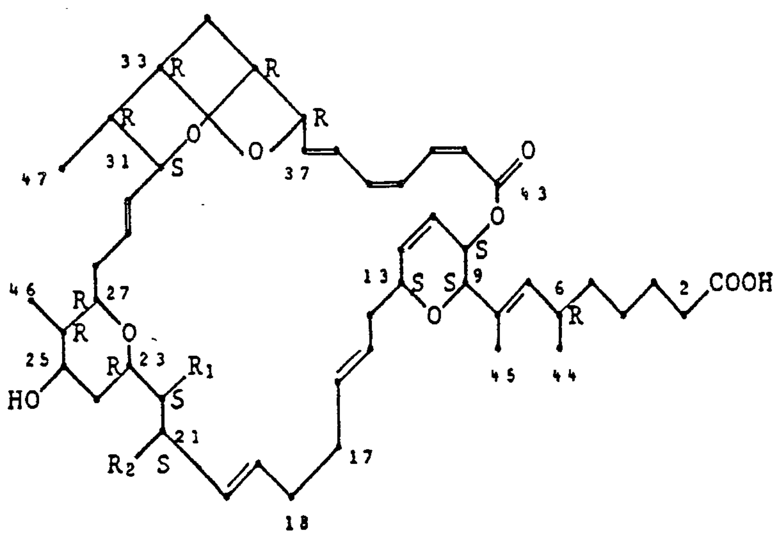

(I) ,

dans laquelle $R_1$ représente l'hydrogène ou un radical hydroxy et $R_2$ un radical hydroxy ou β-glucopyrannosyloxy, et, lorsque $R_1$ désigne un radical hydroxy, l'atome de carbone en 22 a la configuration S, solvates et sels de ces composés.

2. Composé de formule I dans lequel $R_1$ et $R_2$ représentent chacun un radical hydroxy (sorangicine A) et l'atome de carbone en 22 à la configuration S.

3. Solvate d'acétate d'éthyle du composé selon la revendication 2.

4. Composés de formule I dans lesquels $R_1$ représente l'hydrogène et $R_2$ un hydroxy (sorangicine B), ou $R_1$ représente un hydroxy et $R_2$ un radical β-glucopyrannosyloxy (sorangioside A, avec la configuration S du C—22), ou $R_1$ représente l'hydrogène et $R_2$ un radical β-glucopyrannosyloxy (sorangioside B).

5. Procédé pour préparer des composés de formule I, des solvates ou des sels de ceux-ci selon la revendication 1, procédé caractérisé en ce qu'on cultive dans des conditions d'aérobiose la souche So ce 12 (NCIB 12134) de l'espèce Sorangium cellulosum, ou un mutant de cette souche capable de produire des composés de formule I, dans un milieu contenant des sources de carbone et d'azote ainsi que des sels minéraux essentiels, à une température d'environ 20 à 40°C et à un pH d'environ 4,0 à 8,0, et, si on le désire, on isole, éventuellement à l'état d'hydrates, les composés obtenus de formule I, et on transforme un composé obtenu en un sel et/ou un sel obtenu en le composé libre ou en un autre sel.

6. Souche So ce 12 (NCIB 12134) de l'espèce Sorangium cellulosum ou mutant dérivant de cette souche et capable de produire un composé de formule I.

7. Composés doués d'une activité antibiotique qui peuvent être obtenus par le procédé de la revendication 5.

8. Compositions pharmaceutiques qui contiennent un composé de formule I, un solvate ou un sel d'un

tel composé acceptable du point de vue pharmaceutique, selon la revendication 1.

9. Composés de formule I, hydrates ou sels pharmaceutiquement acceptables de ceux-ci, selon la revendication 1, pour l'application dans un procédé de traitement thérapeutique du corps humain ou animal.

10. Composés de formule I, solvates ou sels pharmaceutiquement acceptables de ceux-ci, selon la revendication 1, pour l'application dans la protection des plantes.

11. Application d'un composé de formule I, d'un solvate ou d'un sel pharmaceutiquement acceptable d'un tel composé, selon la revendication 1, pour la préparation d'une composition pharmaceutique.

12. Application d'un composé de formule I, d'un solvate ou d'un sel pharmaceutiquement acceptable d'un tel composé, selon la revendication 1, pour la préparation d'une composition antibactérienne selon la revendication 11.

13. Application d'un composé de formule I, d'un solvate ou d'un sel pharmaceutiquement acceptable d'un tel composé, selon la revendication 1, pour la préparation d'une composition antivirale selon la revendication 11.

14. Application d'un composé de formule I, d'un solvate ou d'un sel d'un tel composé, selon la revendication 1, pour la préparation d'une composition phytosanitaire.

**Claims**

1. Compounds of the formula

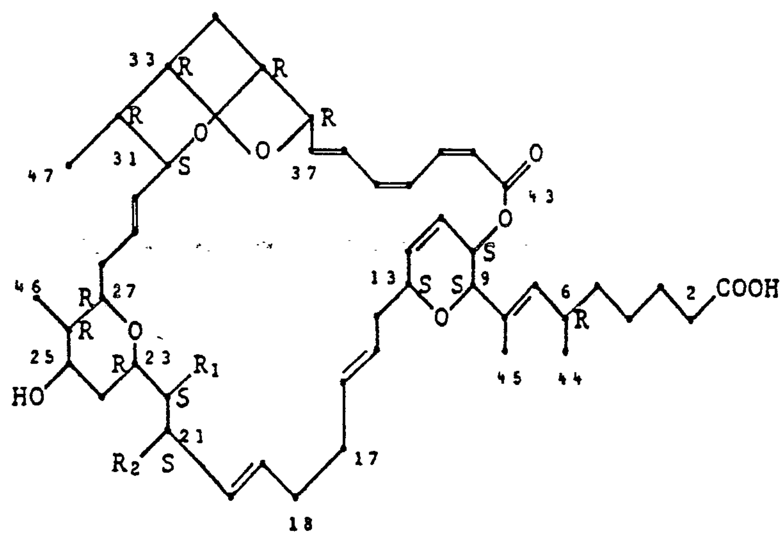

(I) ,

wherein $R_1$ represents hydrogen or hydroxy and $R_2$ represents hydroxy or beta-glucopyranosyloxy and, if $R_1$ represents hydroxy, the C—22 atom has the S-configuration, solvates and salts of these compounds.

2. The compound of the formula I wherein $R_1$ is hydroxy and $R_2$ is hydroxy (sorangicin A) and the C—22 atom has the S-configuration.

3. The ethyl acetate solvate of the compound according to claim 2.

4. The compounds of the formula I wherein $R_1$ is hydrogen, $R_2$ is hydroxy (sorangicin B), $R_1$ is hydroxy and $R_2$ is beta-glucopyranosyloxy (sorangioside A, C—22:S), or $R_1$ is hydrogen and $R_2$ is beta-glucopyranosyloxy (sorangioside B).

5. A process for the preparation of compounds of the formula I, solvates or salts thereof, according to claim 1, characterised in that the strain So ce 12 (NCIB 12134) of the species *Sorangium cellulosum* or a mutant derived from said strain that produces compounds of the formula I is cultured in a culture containing a source of carbon and nitrogen and essential inorganic salts, at approximately from 20° to 40°C and at a pH of approximately from 4.0 to 8.0, under aerobic conditions, and, if desired, the resulting compounds of the formula I are isolated, optionally as hydrates, and a resulting compound is converted into a salt, and/or a resulting salt is converted into the free compound or into another salt.

6. The strain So ce 12 (NCIB 12134) of the species *Sorangium cellulosum* or a mutant derived from said strain, which produces a compound of the formula I.

7. The antibiotically active compounds obtainable by the process according to claim 5.

8. Pharmaceutical preparations containing a compound of the formula I, a solvate or a pharmaceutically acceptable salt thereof, according to claim 1.

9. Compounds of the formula I, hydrates or pharmaceutically acceptable salts thereof, according to claim 1, for use in a method for the therapeutic treatment of the human or animal body.

10. Compounds of the formula I, solvates or pharmaceutically acceptable salts thereof, according to claim 1, for use in plant protection.

11. The use of a compound of formula I, a solvate or a pharmaceutically acceptable salt thereof, according to claim 1, for the production of a pharmaceutical preparation.

12. The use of a compound of the formula I, a solvate or a pharmaceutically acceptable salt thereof, according to claim 1, for the production of an antibacterial preparation according to claim 11.

13. The use of a compound of the formula I, a solvate or a pharmaceutically acceptable salt thereof, according to claim 1, for the production of an antiviral preparation according to claim 11.

14. The use of a compound of the formula I, a solvate or a salt thereof, according to claim 1, for the production of a plant protection preparation.